(19) Europäisches Patentamt
European Patent Office
Office européen des brevets

(11) **EP 2 550 977 B1**

## (12) EUROPEAN PATENT SPECIFICATION

(45) Date of publication and mention
of the grant of the patent:
**28.06.2017 Bulletin 2017/26**

(51) Int Cl.:
***A61L 15/26*** *(2006.01)*    ***A61L 15/42*** *(2006.01)*
***A61L 15/58*** *(2006.01)*

(21) Application number: **12151553.0**

(22) Date of filing: **18.01.2012**

(54) **Polyurethane foam dressing having excellent adhesive properties and manufacturing method thereof**

Polyurethanschaumverband mit ausgezeichneten Hafteigenschaften und Herstellungsverfahren

Compresse en mousse de polyuréthane dotée d'excellentes propriétés adhésives et son procédé de fabrication

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.07.2011 US 201113191648**

(43) Date of publication of application:
**30.01.2013 Bulletin 2013/05**

(73) Proprietor: **Genewel Co., Ltd
Gyeonggi-do (KR)**

(72) Inventors:
• **Kim, Hyun Jung
Gyeonggi-do (KR)**

• **Kim, Kab Keun
410-829 Gyeonggi-do (KR)**
• **Lee, Seung Moon
435-040 Gyeonggi-do (KR)**
• **Park, Il Kyu
410-829 Gyeonggi-do (KR)**

(74) Representative: **Hoffmann Eitle
Patent- und Rechtsanwälte PartmbB
Arabellastraße 30
81925 München (DE)**

(56) References cited:
**WO-A1-93/03105      WO-A1-97/42985
WO-A2-2011/022680      US-A- 3 665 918
US-A- 5 445 604**

EP 2 550 977 B1

**Description**

BACKGROUND OF THE INVENTION

Field of the Invention

The present invention relates to an adhesive polyurethane foam dressing and a manufacturing method thereof, and more particularly to a wound dressing having an adhesive layer comprising an adhesive, a binder and, if necessary, a curing retarder for making an adhesive layer-coating process efficient, and a manufacturing method thereof.

Description of the Prior Art

The skin of the human body is the tissue that covers the body. It protects the body from external chemical and physical influences and performs biochemical functions required for maintaining life. When the skin loses its function due to various wounds, such as traumas, burns or bedsores, the patient will suffer from pain until the wound is completely healed. When the skin is extensively damaged, the patient's life will be endangered.
Thus, many efforts have been made to heal wounds such as traumas, and various healing methods have been developed and are still being developed. In 1962, it was reported that the epithelialization of wounds maintained in a moist state is faster than that in a dry environment. Since then, various studies on wound healing in a moist environment have been reported, and various occlusive dressings for maintaining wounds in a moist environment have been developed and marketed. Also, various wound healing methods based on the concept for maintaining wounds in a moist environment have been developed and applied.
Typical examples of moist environment dressings which are currently known to include film-type dressings, hydrocolloid dressings, hydrogel dressings, and polyurethane foam dressings. Among them, hydrocolloid, hydrogel and polyurethane foam dressings are mainly being used.
Hydrogel-type dressings comprising an impermeable polymeric film layer coated with a hydrogel material are disclosed in US Patent Nos. 5,501,661 and 5,489,262. The polymeric film layer functions to prevent the hydrogel from being dehydrated or dried, while the hydrogel layer comes into contact with the wound bed so as to absorb exudates and maintains a moist wound environment to promote wound healing. However, the hydrogel-type dressings are unsuitable for high-exudate wounds owing to their low moisture permeability and absorption capacity, and when the dressings absorb excessive amounts of exudates, it is difficult for them to be exchanged.
US Patent Nos. 5,503,847 and 5,830,932 disclose hydrocolloid-type dressings that comprise a hydrocolloid layer for relieving external impact and absorbing exudates and a film layer for defending against the invasion of bacteria and impurities. The hydrocolloid-type dressings absorb small amounts of wound exudates to form gel and create a moist environment. Also, the hydrocolloid-type dressings provide a weakly acidic environment for a long period of time in order to prevent tissues from being injured and to promote the growth of cells. However, the hydrocolloid-type dressings are not suitable for healing wounds that produce large quantities of exudates because of their inferior moisture permeability and exudate-absorbing capacity. Furthermore, a portion of the gel is likely to remain on the wound surface when the dressing is changed or removed. Thus, additional work is needed to remove the remaining gel.
US Patent Nos. 5,445,604 and 5,065,752 disclose hydrophilic polyurethane foam dressings having a three layer structure, in which a film is laminated on both sides of a polyurethane foam and in which the film coming into contact with the wound bed (wound contact layer) is mechanically perforated so as to prevent macropores of the wound contact layer from being stuck to the wound bed and to absorb exudates. However, the disclosed dressing is not adhesive to the wound bed, so that a film for attaching and fixing the dressing to the wound bed is required when the dressing is used, and one or more persons are required to attach the dressing, indicating that the dressing has poor handling properties.
In an attempt to solve this problem, US Patent Nos. US 6,051,747 and 6,207,875 disclose hydrophilic polyurethane foam dressings having adhesive properties, in which a porous foam is mechanically perforated to easily absorb exudates and a silicone gel is applied to the wound contact layer to facilitate its adhesion to the wound bed, thus improving handling properties. WO97/42985 relates to a wound dressing comprising a polyurethane foam material coated with a layer of adhesive. The adhesive is applied to the foam and then cured to form a laminate. It includes a silicone gel such as polydimethyl siloxane. However, the disclosed dressing has poor exudate absorption and retention capacities, and thus is unsuitable for application to high-exudate wounds. Also, it needs to be sterilized because of its purpose. However, when it is dealt with by gamma sterilization, its adhesive strength decreases, and when it is dealt with by EO gas sterilization, toxic residues are generated.

## SUMMARY OF THE INVENTION

Accordingly, the present inventors have conducted many studies and experiments to solve the above-described problems occurring in the prior art and, as a result, have reached the present invention. It is an object of the present invention to provide an adhesive polyurethane foam dressing and a manufacturing method thereof, in which the polyurethane foam dressing has high capacities for absorption and retention of exudates so that it can be applied even to high-exudate wounds, and in which an adhesive layer consisting of an adhesive composition is applied to the wound contact layer of the foam dressing so that the foam dressing is advantageous for wound healing, does not show a decrease in adhesive strength even after irradiation with gamma rays and is easily removed without causing pain.

To achieve the above object, the present invention provides a polyurethane foam dressing having excellent adhesive properties, which comprises a laminate of a sponge-like wound contact layer, and a film-like protective layer, in which an adhesive layer having a thickness of 20-95 $\mu$m is formed on the wound contact surface of the wound contact layer, wherein the adhesive layer consists of a hydrophilic adhesive composition comprising 100 parts by weight of a mixture of an acrylate water-soluble adhesive material and a silicone fat-soluble adhesive material as an adhesive and 0.07-5 parts by weight of polyvinyl pyrrolidone based binder. The present invention also provides a method of manufacturing a polyurethane foam dressing having excellent adhesive properties by preparing a polyurethane foam and forming a film-like protective layer and an adhesive layer on the polyurethane foam, wherein the adhesive layer is formed through the steps of: preparing a hydrophilic adhesive composition comprising 100 parts by weight of a mixture of an acrylate water-soluble adhesive material and a silicone fat-soluble adhesive material as an adhesive and 0.07-5 parts by weight of polyvinyl pyrrolidone based binder; and applying the prepared hydrophilic adhesive composition to the polyurethane foam to a predetermined thickness under atmospheric pressure conditions and curing the applied composition under a temperature between 90 °C and 110°C for 3-6 minutes, to produce the polyurethane foam dressing of claim 1.

## DETAILED DESCRIPTION OF THE INVENTION

Hereinafter, the present invention will be described in further detail.

The polyurethane foam dressing having excellent adhesive properties according to the present invention is an adhesive wound dressing comprising an adhesive layer and a foam layer, in which the adhesive layer comprises an adhesive, a binder and, if necessary, acuring retarder, and the foam layer comprises a polyurethane foam.

The polyurethane foam dressing having excellent adhesive properties according to the present invention is preferably a polyurethane foam dressing comprising a laminate of a sponge-like wound contact layer and a film-like protective layer, in which an adhesive layer is formed on the wound contact surface of the wound contact layer, in which the adhesive layer consists of an adhesive composition comprising 100 parts by weight of an adhesive and 0.07-5 parts by weight of a binder.

The film-like protective layer is preferably a film-like polyurethane foam.

The foam layer may be made of a polyurethane foam which is generally known in the art. More preferably, the foam layer has a sponge-like structure comprising a plurality of open cells and pores that make a tunnel from cell to cell. Particularly, the foam layer has a retention capacity of about 300-1,200%, a pore area (membrane opening) of about 10-35% of the total area of the cells, an average open-cell diameter of about 50-300 $\mu$m, an average pore diameter of about 5-85 $\mu$m, an open cell ratio of about 20-70%, a density of about 0.15-0.45 g/cm$^3$, and an absorption capacity of 400-2,000 wt%. Also, the foam layer may have an optional thickness.

The adhesive layer is preferably formed by applying the adhesive composition to the wound contact layer under atmospheric pressure conditions and curing the applied composition.

The adhesive composition may further comprise a curing retarder. The curing retarder is preferably used in an amount ranging from 0.07 to 2 parts by weight based on 100 parts by weight of the adhesive. When the adhesive composition contains the reaction inhibitor within this content range, the curing reaction of the adhesive composition is inhibited before the composition is applied to the wound contact layer, and the curing reaction occurs after the composition has been applied.

The curing retarder is preferably at least one selected from the group consisting of cyclohexanol, methylcyclohexanol, dimethylcyclohexanol, and ethynylcyclohexanol.

The curing retarder is a mixture of two compounds selected from among the above listed compounds, which are preferably mixed at a ratio of 1:0.1 to 0.1:1, and more preferably 1: 0.8 to 0.8: 1.

The adhesive strength (gf/12 mm at breakage) of the adhesive layer after irradiation with gamma rays is preferably 80% or higher, and more preferably 90% or higher of the adhesive strength (gf/12 mm at breakage) before irradiation with gamma rays.

The adhesive layer preferably has a thickness of 20-95 $\mu$m.

The adhesive preferably consists of a mixture of a water-soluble adhesive material and a fat-soluble adhesive material.

The weight ratio between the water-soluble adhesive material and the fat-soluble adhesive material (fat-soluble adhesive material: water-soluble adhesive material) is in the range of 1.0:0.01 to 1.0:0.4, and more preferably 1.0:0.15 to 1.0:0.25. In this mixing range, the adhesive materials are uniformly mixed with each other and are highly compatible with the polyurethane foam.

The binder serves to enhance the phase stability of the adhesive, and to bind the water-soluble adhesive material to the fat-soluble adhesive material, and also to bind the water-soluble adhesive material to the polyurethane foam. The binder may be used in an amount of 0.07-5 parts by weight, and preferably 0.7-3 parts by weight, based on 100 parts by weight of the hydrophilic adhesive. When the binder is used in this amount, it shows excellent phase stability, binding properties and processing properties.

The binder is preferably a polyvinyl pyrrolidone compound, and more preferably one or more selected from the group consisting of PVP 12CF, PVP CL, PVP CL-F, PVP 25, PVP 30, PVP 90-F and PVP VA64, but is not limited thereto.

The water-soluble adhesive material serves to provide compatibility between the adhesive layer and the polyurethane foam layer and is preferably at least one polymer resin selected from the group consisting of methylacrylate, ethylacrylate, octylacrylate, ethylhexylacrylate, butylmethacrylate, acrylic acid, and methacrylic acid. These polymer resins that are used as the water-soluble adhesive material may be used alone or in a mixture of two or more.

The fat-soluble adhesive material serves to provide stable adhesion of the foam dressing to the skin. It may be a silicone compound and is preferably at least one selected from the group consisting of a polydimethylsiloxane with vinyl groups, and a hydrogen-terminated dimethylsiloxane.

The fat-soluble adhesive material is preferably a mixture of a polydimethylsiloxane having vinyl groups, and a hydrogen-terminated dimethylsiloxane, which are mixed at a weight ratio of 1:0.1 to 0.1:1.

Specifically, the fat-soluble adhesive material may be a soft skin adhesive (SSA) commercially available from, for example, Dow Corning, Wacker, BLUESTAR Silicone Co., Ltd., and examples of SSA commercially available from Dow Corning include MG 7-9700, MG 7-9800, MG 7-9850, MG 7-9900 and the like.

Also, the present invention relates to a method of manufacturing a polyurethane foam dressing by preparing a polyurethane foam and forming a film-like protective layer and an adhesive layer on the polyurethane foam, in which the adhesive layer is formed through the steps of: preparing an adhesive composition comprising 100 parts by weight of an adhesive and 0.07-5 parts by weight of a binder; and applying the prepared adhesive composition to the polyurethane foam to a predetermined thickness under atmospheric pressure conditions and curing the applied composition.

As used herein, the term "application under atmospheric pressure conditions" means that a roller, for example, is not used to apply pressure to the adhesive composition during or immediately after application of the composition.

The adhesive composition may be prepared by mixing the adhesive, the binder and, if necessary, the curing retarder.

Applying the dhesive composition can be performed using a thin film applicator.

The polyurethane foam preferably has a thickness of 1.0-10 mm. In this thickness range, the dressing is easily attached and detached during the use thereof.

The polyurethane foam preferably has a density of 0.1-1.0 g/cm$^3$. In this density range, the polyurethane foam dressing has high absorption and retention capacities and low hardness, so that it is removed from the skin without causing pain.

The adhesive layer formed by applying the adhesive composition to the polyurethane foam has a post-cure thickness of 20-95 $\mu$m. If the post-cure thickness of the adhesive layer is less than 20 $\mu$m, the adhesive layer will be easily separated from the foam layer and will be difficult to prepare, and if the post-cure thickness is more than 95 $\mu$m, the adhesive layer will completely seal the foam layer so as to reduce the absorption capacity of the foam layer.

The curing of the applied adhesive composition is preferably carried out at a temperature between 90°C and 110 °C for 3-6 minutes. Under these conditions, the curing of the composition is efficiently achieved.

The curing process may be carried out in an oven.

Hereinafter, the present invention will be described in further detail with reference to examples, but the scope of the present invention is not limited to these examples.

Examples

Examples 1 to 6, Reference Examples 1 to 2

10 parts by weight of water-soluble polyacrylic acid (GME 2397; Medical grade; manufactured by Cytec Industries Inc.), 50 parts by weight of two-component silicone A (MG 7-9700, Dow Corning), 50 parts by weight of two-component silicone B (MG 7-9700, Dow Corning) and 2 parts by weight of a binder (polyvinyl pyrrolidone; S630; manufactured by BASF) were mixed with each other, thus preparing an adhesive composition.

The prepared adhesive composition was applied to a release film in such a manner that the post-cure thickness thereof was 10, 20, 40, 50, 60, 80, 95 and 110 $\mu$m (Reference Example 1, Examples 1 to 6, Reference Example 2 respectively). A urethane foam (thickness: 2.0mm, and density: 0.2 g/cm$^3$) was applied to each of the release films applied with the adhesive composition, after which the applied composition was cured at 100 °C for 5 minutes, thus manufacturing adhesive wound dressings (adhesive polyurethane dressings).

The properties of the prepared adhesive wound dressings (Examples 1 to 6, Reference Examples 1 to 2) were measured according to the methods described in the following test example, and the results of the measurement are shown in Table 1 below.

Examples 7 to 12; and Reference Examples 3 to 4

Adhesive wound dressings (Reference Example 3, Examples 7 to 12, Reference Example 4 respectively) were manufactured in the same manner as Example 1, except that 20 parts by weight of polyacrylic acid was used.

The properties of the prepared adhesive wound dressings (Example 7 to 12, Reference Examples 3 to 4) were measured according to the methods described in the following test example, and the results of the measurement are shown in Table 2 below.

Comparative Example 1: Foam alone

A polyurethane foam having no adhesive layer was used.

The properties of the polyurethane foam were measured according to the methods described in the following test example, and the results of the measurement are shown in Tables 1 and 2 below.

Comparative Example 2: Formation of adhesive layer consisting of water-soluble adhesive material alone

An adhesive wound dressing was manufactured in the same manner as Example 1, except that water-soluble polyacrylic acid alone, a water-soluble adhesive material, was used instead of the adhesive composition such that the post-cure thickness of the formed adhesive layer was 40 $\mu$m.

The properties of the manufactured adhesive wound dressing were measured according to the methods described in the following test example, and the results of the measurement are shown in Tables 1 and 2 below.

Comparative Example 3: Formation of adhesive layer consisting of fat-soluble adhesive material alone

An adhesive wound dressing was manufactured in the same manner as Example 1, except that silicone (MG 7-9700, Dow Corning) alone, a fat-soluble adhesive material, was used instead of the adhesive composition such that the post-cure thickness of the formed adhesive layer was 40 $\mu$m.

The properties of the manufactured adhesive wound dressing were measured according to the methods described in the following test example, and the results of the measurement are shown in Tables 1 and 2 below.

Comparative Example 4: Mepilex

Mepilex, a polyurethane foam dressing coated with a silicone adhesive material, was used.

The properties of the product Mepilex were measured according to the methods described in the following test example, and the results of the measurement are shown in Tables 1 and 2 below.

Test Example

Surface analysis (SEM) of adhesive wound dressings

In order to examine the adhesive layer of the adhesive foam dressings of Examples 1 to 12 and Comparative Examples 1 to 4, Reference Examples 1 to 4, surface analysis was carried out.

For surface analysis, each of the adhesive wound dressings was coated with platinum ions and then placed on a scanning electron microscope (SHIMADZU. Co. Ltd, SUPERSCAN SS-550), after which the sample of each of Examples 1 to 12, Reference Examples 1 to 4, and Comparative Examples 1 to 4 was examined.

Comparison of adhesive strength between adhesive wound dressings

In order to examine the adhesive strength of the adhesive dressings of Examples 1 to 12, Reference Example 1 to 4 and Comparative Examples 1 to 4, each of the prepared samples was placed on an Instron universal tester, and the adhesive strength thereof was tested in accordance with ASTM D3330. The test for the adhesive strength of the adhesive wound dressings of Examples 1 to 12, Reference Examples 1 to 4 and Comparative Examples 1 to 4 was carried out for two sample groups: a sample group irradiated with 25 KGY of gamma rays; and a sample group not irradiated with gamma rays.

Specifically, each of samples prepared to have a size of 1.2 cm X 15 cm was placed and fixed between grips, after which the adhesive strength of each sample was measured at a crosshead speed of 300 mm/min, and the maximum stress was determined.

The results of the measurement are shown in Tables 1 and 2 below.

Initial absorption speed of adhesive wound dressings

In order to measure the initial absorption speed of Examples 1 to 12, Reference Examples 1 to 4 and Comparative Examples 1 to 4, PBS was dropped from a height of 1 cm onto the wound contact surface of the dressings, and the time taken for the PBS to be absorbed into the dressing was measured. The results of the measurement are shown in Tables 1 and 2 below. The test for the initial absorption speed of the adhesive wound dressings of Examples 1 to 12, Reference Examples 1 to 4 and Comparative Examples 1 to 4 was carried out for two sample groups: a sample group irradiated with 25 KGY of gamma rays; and a sample group not irradiated with gamma rays.

Measurement of water absorption rate and water retention rate of adhesive wound dressings

To measure the water absorption rate of the adhesive foam dressings of Examples 1 to 12, Reference Examples 1 to 4 and Comparative Examples 1 to 4, each of the adhesive wound dressings was cut to a size of 3 cm x 3 cm and dried in a vacuum oven at 50 °C for 24 hours, after which the initial weight of the cut sample was measured (A). Then, the cut sample was immersed in distilled water at 25 °C for 24 hours, after which the moisture on the surface of the sample was removed using dust-free paper, and then the weight of the sample was measured (B). Then, the water absorption rate of the sample was calculated using the following equation 1. The test for the water absorption rate and water retention rate of the adhesive wound dressings of Examples 1 to 12, Reference Example 1 to 4 and Comparative Examples 1 to 4 was carried out for two sample groups: a sample group irradiated with 25 KGY of gamma rays; and a sample group not irradiated with gamma rays.

$$[\text{Equation 1}]$$

$$\text{Water absorption rate (\%)} = (B-A)/A \times 100$$

To determine the water retention rate of the adhesive polyurethane dressings, each of the adhesive wound dressings was cut to a size of 3 cm x 3 cm and dried in a vacuum oven at 50 °C for 24 hours, after which and the initial weight of the cut sample was measured (A). Then, the sample was immersed in distilled water at 25 °C for 24 hours, after which a 3-kg roller was rolled three times on the sample, and then the weight of the sample was measured (C). Then, the water retention rate of the sample was calculated according to the following equation 2:

$$[\text{Equation 2}]$$

$$\text{Water retention rate (\%)} = (C-A)/A \times 100$$

[Table 1]

| Physical properties | | Comp. Example 1 | Reference | Examples | | | | | | | Reference Example | Comp. Example 2 | Comp. Example 3 | Comp. Example 4 |
|---|---|---|---|---|---|---|---|---|---|---|---|---|---|---|
| | | | 1 | 1 | 2 | 3 | 4 | 5 | 6 | 2 | | | |
| Thickness of adhesive layer | | 40 μm | 10 μm | 20 μm | 40 μm | 50 μm | 60 μm | 80 μm | 95 μm | 110 μm | 40 μm | 40 μm | 110 μm |
| pH (distilled water pH 7.54) | | 7.31 | 6.88 | 6.93 | 6.93 | 6.88 | 6.89 | 6.91 | 6.87 | 6.92 | 6.92 | 6.92 | 6.9 |
| Adhesive strength (gf/12 mm at breakage) | Not irradiated | ND | 4 | 9 | 9 | 10 | 10 | 10 | 11 | 12 | ND | Silicone layer was separated from foam layer | 14 |
| | Irradiated with gamma rays | ND | 4 | 9 | 9 | 9 | 10 | 10 | 10 | 11 | ND | Silicone layer was separated from foam layer | 5 |
| Initial absorption speed (sec) | Not irradiated | 3 | 15 | 15 | 15 | 16 | 16 | 17 | 17 | 55 | 4 | 16 | 185 |
| | Irradiated with gamma rays | 3 | 15 | 16 | 15 | 15 | 16 | 17 | 17 | 51 | 4 | 16 | 196 |
| Water retention | Not irradiated | 1.18 | 1.19 | 1.17 | 1.17 | 1.16 | 1.18 | 1.19 | 1.16 | 1.14 | 1.15 | 1.14 | 0.24 |
| | Irradiated with gamma rays | 1.18 | 1.18 | 1.17 | 1.18 | 1.16 | 1.18 | 1.18 | 1,17 | 1.15 | 1.15 | 1.13 | 0.14 |
| Water absorption | Not irradiated | 2.47 | 2.42 | 2.42 | 2.42 | 2.39 | 2.40 | 2.43 | 2.40 | 2.39 | 2.40 | 2.40 | 1.68 |
| | Irradiated with gamma rays | 2.47 | 2.43 | 2.44 | 2.43 | 2.4 | 2.39 | 2.42 | 2.41 | 2.38 | 2.41 | 2.39 | 0.44 |

[Table 2]

| Physical properties | | Comp. Example 1 | Reference Example 3 | Examples 7 | 8 | 9 | 10 | 11 | 12 | Reference Example 4 | Comp. Example 2 | Comp. Example 3 | Comp. Example 4 |
|---|---|---|---|---|---|---|---|---|---|---|---|---|---|
| pH (distilled water pH 7.54) | | 7.31 | 6.77 | 6.75 | 6.75 | 6.66 | 6.94 | 6.83 | 6.79 | 6.78 | 6.88 | 6.72 | 6.9 |
| Thickness of adhesive layer | | 40 μm | 10 μm | 20 μm | 40 μm | 50 μm | 60 μm | 80 μm | 95 μm | 110 μm | 40 μm | 40 μm | 110 μm |
| Adhesive strength (gf/12 mm at breakage) | Not irradiated | ND | 4 | 11 | 11 | 11 | 13 | 13 | 14 | 15 | ND | Silicone layer was separated from foam layer | 14 |
| | Irradiated with gamma rays | ND | 4 | 11 | 10 | 11 | 13 | 13 | 14 | 14 | Silicone layer was separated from foam layer | Silicone layer was separated from foam layer | 5 |
| Initial absorption speed (sec) | Not irradiated | 3 | 15 | 15 | 15 | 16 | 16 | 17 | 17 | 55 | 4 | 16 | 185 |
| | Irradiated with gamma rays | 3 | 15 | 16 | 16 | 15 | 15 | 17 | 16 | 52 | 4 | 15 | 196 |
| Water retention | Not irradiated | 1.19 | 1.17 | 1.18 | 1.17 | 1.19 | 1.16 | 1.18 | 1.17 | 1.19 | 1.15 | 1.15 | 0.24 |
| | Irradiated with gamma rays | 1.18 | 1.18 | 1.18 | 1.18 | 1.17 | 1.17 | 1.18 | 1.18 | 1.19 | 1.15 | 1.14 | 0.14 |
| Water absorption | Not irradiated | 2.47 | 2.40 | 2.41 | 2.41 | 2.38 | 2.44 | 2.39 | 2.38 | 2.40 | 2.40 | 2.38 | 1.68 |
| | Irradiated with gamma rays | 2.47 | 2.41 | 2.42 | 2.43 | 2.39 | 2.41 | 2.4 | 2.37 | 2.41 | 2.40 | 2.39 | 0.44 |

As can be seen in Table 1 and 2 above, the adhesive strength of the samples was measured for each of the samples having different adhesive layer thicknesses, the samples having different contents of aqueous polyacrylic acid (water-soluble adhesive material), the samples irradiated with gamma rays, and the samples not irradiated with gamma rays. As the thickness of the adhesive layer increased, the adhesive strength slightly increased. Also, there was no difference in adhesive strength between the contents of the adhesive materials. In addition, the samples prepared in Examples 1 to 12 did not show a decrease in adhesive strength even after they had been irradiated with gamma rays. However, the adhesive strength of the product of Comparative Example 4 decreased from 14 gf/12mm before gamma ray irradiation to 5 gf/12mm after gamma ray irradiation. Also, where the thickness of the adhesive layer was 10 $\mu$m (Reference Examples 1 and 3), there were problems in that the adhesive strength was low and the adhesive layer was easily separated from the foam layer. Where the thickness of the adhesive layer was 110 $\mu$m (Reference Examples 2 and 4), there was a problem in that the initial absorption speed was slow. In Comparative Example 2, the polyacrylic acid was absorbed into the foam, thus making it difficult to measure the adhesive strength of the sample. Meanwhile, the initial absorption speed, water absorption rate and water retention rate of the sample of Comparative Example 4 were lower than those of Examples 1 to 12 and Comparative Examples 1 to 3 before gamma ray irradiation and were further reduced after gamma ray irradiation.

Examples 13 to 18 and Reference Example 5: Examination of curing time upon addition of reaction inhibitor

A reaction inhibitor (PT88, manufactured by Waker Corp.) was added to the adhesive composition of Example 1 in varying amounts of 0.1, 0.3, 0.4, 0.5, 0.6, 0.7 and 3 parts by weight, after which each of the mixture solutions was sufficiently stirred for 1 hour, thereby preparing adhesive compositions containing the reaction inhibitor (Examples 13 to 18, Reference Example 5).

Each of the adhesive compositions containing the reaction inhibitor was maintained at 100 °C for 5 minutes while whether the compositions were cured was examined. The results of the examination are shown in Table 3 below.

[Table 3]

| | Examples | | | | | | Reference Example |
|---|---|---|---|---|---|---|---|
| | 13 | 14 | 15 | 16 | 17 | 18 | 5 |
| Content of reaction inhibitor | 0.1 | 0.3 | 0.4 | 0.5 | 0.6 | 0.7 | 3 |
| Cured (O, X) | O | O | O | O | O | O | X |

As can be seen in Table 3 above, the samples of Examples 13 to 18 were all cured when they were maintained at 100 °C for 5 minutes, but the sample of Reference Example 5 was not cured even when it was maintained at 100 °C for 5 minutes.

Examples 19 to 20; Reference Examples 6 to 7: Selection of content of acrylic adhesive

To 50 parts by weight of 2-component silicone A (Dow Corning MG 7-9700) and 50 parts by weight of 2-component silicone B (Dow Corning MG 7-9700), polyacrylic acid (GME 2397; Medical grade; manufactured by Cytec Industries Corp.), a water-soluble adhesive material, was added in varying amounts of 5, 10, 20 and 30 parts by weight. Then, the mixtures were sufficiently stirred for 30 minutes, after which they were sufficiently defoamed in a defoaming machine for 1 hour, thereby preparing adhesive compositions (Examples 19 to 20, Reference Examples 6 to 7, respectively).

To manufacture adhesive wound dressings, a polyurethane foam (thickness: 2.0 mm; and density: 0.2 g/cm$^3$) was prepared, and each of the prepared adhesive compositions was applied to a release film (easily separable from the silicone adhesive layer) to a thickness of 50 $\mu$m. The polyurethane foam (thickness: 2.0 mm; and density: 0.2 g/cm$^3$) was applied to the film applied with each of the adhesive compositions, after which each of the adhesive compositions was cured at 100 °C for 5 minutes, thereby manufacturing adhesive wound dressings (adhesive polyurethane dressings) comprising an adhesive layer consisting of each of the adhesive compositions.

Among the manufactured adhesive wound dressings, in the case when the content of polyacrylic acid (water-soluble adhesive material) was 5 parts by weight (Reference Example 6), the adhesive layer was easily separated from the foam layer in comparison with the cases when the content of polyacrylic acid was 10 parts by weight and 20 parts by weight (Examples 11 and 20). Also, in the case when the content of polyacrylic acid was 30 parts by weight (Reference Example 7), the compatibility of the polyacrylic acid with the silicone (fat-soluble adhesive material) was somewhat reduced.

Examples 21 to 28: Test for kinds and conditions of applicable foams

Adhesive wound dressings (Examples 21 to 28, respectively) were manufactured in the same manner as Example 1, except that urethane foams having various thicknesses of 0.5, 1.0, 2.0, 3.0, 4.0, 5.0, 10 and 20 mm and a density of 0.2 g/cm$^3$ were used.

As a result, it could be seen that the adhesive wound dressings could be easily manufactured regardless of the thickness and density of the urethane foam and were applied as products without any problem. Also, it could be seen that all the products were easily removed from the portion (skin) to which they have been applied.

As described above, according to the present invention, the adhesive polyurethane foam dressing for wound healing can be easily manufactured. The adhesive polyurethane foam dressing manufactured according to the present invention has excellent capacities for absorption and retention of wound exudates and are easily applied to wounds due to their adhesive properties. In addition, the adhesive polyurethane foam dressing according to the present invention shows no decrease in adhesive strength even after gamma ray irradiation and can be removed without causing pain.

**Claims**

1. A polyurethane foam dressing having excellent adhesive properties, which comprises a laminate of a sponge-like wound contact layer, and a film-like protective layer, in which an adhesive layer having a thickness of 20-95 μm is formed on the wound contact surface of the wound contact layer,
   wherein the adhesive layer consists of a hydrophilic adhesive composition comprising 100 parts by weight of a mixture of an acrylate water-soluble adhesive material and a silicone fat-soluble adhesive material as an adhesive and 0.07-5 parts by weight of polyvinyl pyrrolidone based binder.

2. The polyurethane foam dressing of claim 1, wherein the adhesive layer is formed by applying the hydrophilic adhesive composition on the wound contact layer under atmospheric pressure conditions and curing the applied composition.

3. The polyurethane foam dressing of claim 1, wherein the adhesive composition further comprises a curing retarder.

4. The polyurethane foam dressing of claim 3, wherein the curing retarder is contained in an amount of 0.07-2 parts by weight based on 100 parts by weight of the adhesive.

5. The polyurethane foam dressing of claim 1, wherein the adhesive strength (gf/12mm at breakage) of the adhesive layer after irradiation with gamma rays is more than 80% of the adhesive strength (gf/12mm at breakage) before irradiation with gamma rays.

6. The polyurethane foam dressing of claim 1, wherein the weight ratio of the silicone fat-soluble adhesive material: the acrylate water-soluble adhesive material is 1:0.01 to 1:0.4.

7. The polyurethane foam dressing of claim 1, wherein the acrylate water-soluble adhesive material is at least one polymer resin consisting of groups selected from methylacrylate, ethylacrylate, octylacrylate, ethylhexylacrylate, butylmethacrylate, acrylic acid, and methacrylic acid.

8. The polyurethane foam dressing of claim 1, wherein the silicone fat-soluble adhesive material is at least one selected from the group consisting of a polydimethylsiloxane with vinyl groups, and a hydrogen-terminated dimethylsiloxane.

9. The polyurethane foam dressing of claim 3, wherein the curing retarder is at least one selected from the group consisting of cyclohexanol, methylcyclohexanol, dimethylcyclohexanol, and ethynylcyclohexanol.

10. A method of manufacturing a polyurethane foam dressing having excellent adhesive properties by preparing a polyurethane foam and forming a film-like protective layer and an adhesive layer on the polyurethane foam,
    wherein the adhesive layer is formed through the steps of: preparing a hydrophilic adhesive composition comprising 100 parts by weight of a mixture of an acrylate water-soluble adhesive material and a silicone fat-soluble adhesive material as an adhesive and 0.07-5 parts by weight of polyvinyl pyrrolidone based binder; and applying the prepared hydrophilic adhesive composition to the polyurethane foam to a predetermined thickness under atmospheric pressure conditions and curing the applied composition under a temperature between 90 °C and 110°C for 3-6 minutes, to produce the polyurethane foam dressing of claim 1.

**11.** The method of claim 10, wherein the adhesive composition further comprises a curing retarder in an amount of 0.07-2 parts by weight based on 100 parts by weight of the adhesive.

**12.** The method of claim 10, further comprising a step of irradiation the produced polyurethane foam dressing with gamma rays.

**Patentansprüche**

**1.** Polyurethanschaumverband mit ausgezeichneten Hafteigenschaften, welcher ein Laminat aus einer schwammartigen Wundkontaktschicht und einer folienartigen Schutzschicht umfasst, worin eine Haftschicht mit einer Dicke von 20-95 µm auf der Wundkontaktoberfläche der Wundkontaktschicht gebildet ist,
wobei die Haftschicht aus einer hydrophilen Haftzusammensetzung besteht, die 100 Gewichtsanteile einer Mischung eines wasserlöslichen Acrylat-Haftmaterials und eines fettlöslichen Silikon-Haftmaterials als ein Haftmittel sowie 0,07-5 Gewichtsanteile eines Polyvinylpyrrolidonbasierten Bindemittels umfasst.

**2.** Polyurethanschaumverband gemäß Anspruch 1, wobei die Haftschicht durch Auftragen der hydrophilen Haftzusammensetzung auf die Wundkontaktschicht unter Atmosphärendruckbedingungen und Aushärtenlassen der aufgetragenen Zusammensetzung gebildet wird.

**3.** Polyurethanschaumverband gemäß Anspruch 1, wobei die Haftzusammensetzung ferner einen Aushärtungsverzögerer umfasst.

**4.** Polyurethanschaumverband gemäß Anspruch 3, wobei der Aushärtungsverzögerer bei einem Gehalt von 0,07-2 Gewichtsanteilen, basierend auf 100 Gewichtsanteilen des Haftmittels, enthalten ist.

**5.** Polyurethanschaumverband gemäß Anspruch 1, wobei die Haftfestigkeit (gf/12 mm bei Bruch) der Haftschicht nach Bestrahlung mit Gammastrahlen mehr als 80% der Haftfestigkeit (gf/12 mm bei Bruch) vor Bestrahlung mit Gammastrahlen beträgt.

**6.** Polyurethanschaumverband gemäß Anspruch 1, wobei das Gewichtsverhältnis des fettlöslichen Silikon-Haftmaterials zum wasserlöslichen Acrylat-Haftmaterial 1:0,01 bis 1:0,4 beträgt.

**7.** Polyurethanschaumverband gemäß Anspruch 1, wobei das wasserlösliche Acrylat-Haftmaterial wenigstens ein Polymerharz, bestehend aus Gruppen, ausgewählt aus Methylacrylat, Ethylacrylat, Octylacrylat, Ethylhexylacrylat, Butylmethacrylat, Acrylsäure und Methacrylsäure, ist.

**8.** Polyurethanschaumverband gemäß Anspruch 1, wobei das fettlösliche Silikon-Haftmaterial wenigstens eines, ausgewählt aus der Gruppe, bestehend aus: Polydimethylsiloxan, das Vinylgruppen aufweist, und einem Wasserstoffterminierten Dimethylsiloxan, ist.

**9.** Polyurethanschaumverband gemäß Anspruch 3, wobei der Aushärtungsverzögerer wenigstens einer, ausgewählt aus der Gruppe, bestehend aus Cyclohexanol, Methylcyclohexanol, Dimethylcyclohexanol und Ethinylcyclohexanol, ist.

**10.** Verfahren zur Herstellung eines Polyurethanschaumverbands mit ausgezeichneten Hafteigenschaften durch Zubereiten eines Polyurethanschaums und Bilden einer folienartigen Schutzschicht und einer Haftschicht auf dem Polyurethanschaum,
wobei die Haftschicht durch die Schritte gebildet wird: Zubereiten einer hydrophilen Haftzusammensetzung, die 100 Gewichtsanteile einer Mischung eines wasserlöslichen Acrylat-Haftmaterials und eines fettlöslichen Silikon-Haftmaterials als ein Haftmittel und 0,07-5 Gewichtsanteile eines Polyvinylpyrrolidonbasierten Bindemittels umfasst; und Auftragen der zubereiteten hydrophilen Haftlösung auf den Polyurethanschaum bis zu einer vorgegebenen Dicke unter Atmosphärendruckbedingungen und Aushärtenlassen der aufgetragenen Zusammensetzung unter einer Temperatur zwischen 90 °C und 110 °C für 3-6 Minuten, so dass der Polyurethanschaumverband gemäß Anspruch 1 hergestellt wird.

**11.** Verfahren gemäß Anspruch 10, wobei die Haftzusammensetzung ferner einen Aushärtungsverzögerer bei einem Gehalt von 0,07-2 Gewichtsanteilen, basierend auf 100 Gewichtsanteilen des Haftmittels, umfasst.

**12.** Verfahren gemäß Anspruch 10, ferner umfassend einen Schritt der Bestrahlung des hergestellten Polyurethanschaumverbands mit Gammastrahlen.


**Revendications**

**1.** Pansement en mousse de polyuréthane ayant d'excellentes propriétés adhésives, qui comprend un stratifié d'une couche de contact avec une plaie de type éponge, et d'une couche protectrice de type film, dans lequel une couche adhésive ayant une épaisseur de 20 à 95 $\mu$m est formée sur la surface de contact avec une plaie de la couche de contact avec une plaie,
dans lequel la couche adhésive consiste en une composition adhésive hydrophile comprenant 100 parties en poids d'un mélange d'un matériau adhésif soluble dans l'eau à base d'acrylate et d'un matériau adhésif soluble dans une matière grasse à base de silicone en tant qu'adhésif et 0,07 à 5 parties en poids d'un liant à base de polyvinylpyrrolidone.

**2.** Pansement en mousse de polyuréthane selon la revendication 1, dans lequel la couche adhésive est formée par application de la composition adhésive hydrophile sur la couche en contact avec la plaie dans des conditions de pression atmosphérique et séchage de la composition appliquée.

**3.** Pansement en mousse de polyuréthane selon la revendication 1, dans lequel la composition adhésive comprend en outre un ralentisseur de séchage.

**4.** Pansement en mousse de polyuréthane selon la revendication 3, dans lequel le ralentisseur de séchage est contenu en une quantité de 0,07 à 2 partie en poids sur la base de 100 parties en poids de l'adhésif.

**5.** Pansement en mousse de polyuréthane selon la revendication 1, dans lequel la force adhésive (gf/12 mm à la rupture) de la couche adhésive après irradiation avec des rayons gamma est plus de 80 % de la force adhésive (gf/12 mm à la rupture) avant irradiation avec des rayons gamma.

**6.** Pansement en mousse de polyuréthane selon la revendication 1, dans lequel le rapport de poids du matériau adhésif soluble dans une matière grasse à base de silicone : le matériau adhésif soluble dans l'eau à base d'acrylate est 1 : 0,01 à 1 : 0,4.

**7.** Pansement en mousse de polyuréthane selon la revendication 1, dans lequel le matériau adhésif soluble dans l'eau à base d'acrylate est au moins une résine de polymère constituée de groupes sélectionnés parmi l'acrylate de méthyle, l'acrylate d'éthyle, l'acrylate d'octyle, l'acrylate d'éthylhexyle, le méthacrylate de butyle, l'acide acrylique, et l'acide méthacrylique.

**8.** Pansement en mousse de polyuréthane selon la revendication 1, dans lequel le matériau adhésif soluble dans une matière grasse à base de silicone est au moins un sélectionné dans le groupe constitué d'un polydiméthysiloxane avec des groupes vinyle, et d'un diméthylsiloxane à terminaison hydrogène.

**9.** Pansement en mousse de polyuréthane selon la revendication 3, dans lequel le ralentisseur de séchage est au moins un sélectionné dans le groupe constitué du cyclohexanol, du méthylcyclohexanol, du diméthylcyclohexanol, et de l'éthynylcyclohexanol.

**10.** Procédé de fabrication d'un pansement en mousse de polyuréthane ayant d'excellentes propriétés adhésives par préparation d'une mousse de polyuréthane et formation d'une couche protectrice de type film et d'une couche adhésive sur la mousse de polyuréthane,
dans lequel la couche adhésive est formée par les étapes de : préparer une composition adhésive hydrophile comprenant 100 parties en poids d'un mélange d'un matériau adhésif soluble dans l'eau à base d'acrylate et d'un matériau adhésif soluble dans une matière grasse à base de silicone en tant qu'adhésif et 0,07 à 5 parties en poids de liant à base de polyvinylpyrrolidone ; et appliquer la composition adhésive hydrophile préparée sur la mousse de polyuréthane à une épaisseur prédéterminée dans des conditions de pression atmosphérique et sécher la composition appliquée à une température entre 90°C et 110°C pendant 3 à 6 minutes, pour préparer le pansement en mousse de polyuréthane selon la revendication 1.

**11.** Procédé selon la revendication 10, dans lequel la composition adhésive comprend en outre un ralentisseur de séchage en une quantité de 0,07 à 2 parties en poids sur la base de 100 parties en poids de l'adhésif.

12. Procédé selon la revendication 10, comprenant en outre une étape d'irradiation du pansement en mousse de polyuréthane produit avec des rayons gamma.

**EP 2 550 977 B1**

### Patent documents cited in the description

- US 5501661 A
- US 5489262 A
- US 5503847 A
- US 5830932 A
- US 5445604 A
- US 5065752 A
- US 6051747 A
- US 6207875 B
- WO 9742985 A